# EUROPÄISCHE PATENTSCHRIFT

(11) **EP 3 696 553 B1**
(45) Veröffentlichungstag und Bekanntmachung des Hinweises auf die Patenterteilung: **06.10.2021**
(21) Anmeldenummer: 19156762.7
(22) Anmeldetag: 12.02.2019
(51) Int. Cl.: G01N 35/10, B01L 3/00, A61B 5/15

(54) **HOHLNADEL FÜR EINEN PROBENPIPETTOR**
HOLLOW NEEDLE FOR A SAMPLE PIPETTOR
AIGUILLE CREUSE POUR UN PIPETEUR D'ÉCHANTILLONS

(43) Veröffentlichungstag der Anmeldung: 19.08.2020
(73) Patentinhaber: TECAN TRADING AG, 8708 Männedorf (CH)
(72) Erfinder: WÜRGLER, ROLAND, 8708 Männedorf (CH); KÜMMERLI, PETER, 3066 Stettlen (CH); ERB, DOMINIC, 8400 Winterthur (CH); TANASESCU, PAUL, 8045 Zürich (CH)
(74) Vertreter: Troesch Scheidegger Werner AG

(56) Entgegenhaltungen:
- EP-A1- 2 711 080
- WO-A1-2009/024522
- DE-A1- 19 905 644

## Beschreibung

### TECHNISCHES GEBIET

Die vorliegende Erfindung betrifft eine Hohlnadel für einen Probenpipettor in einem Gerät zur Laborautomatisierung, insbesondere zur Entnahme von Probenflüssigkeit aus einem verschlossenen Probengefäss, wobei zur Entnahme der Probenflüssigkeit der Verschluss des Probengefässes mit der Hohlnadel durchstossen wird. Probenflüssigkeiten können auch in Probengefässe mit Verschluss abgegeben werden, wobei die Hohlnadel vor der Abgabe der Probenflüssigkeit den Verschluss durchstösst. Unter einem Gerät zur Laborautomatisierung kann beispielsweise ein Pipettierroboter oder ein Autosampler für Chromatographen oder Spektrometer verstanden werden. Unter Probengefäss können Behälter verstanden werden, die Analyten, Lösungen von Analyten oder biologische Flüssigkeiten enthalten oder die Reagenzien enthalten. Unter Verschluss können ein Verschluss mit eingebauter Kunststoff- oder Gummimembran oder ein Kunststoff- oder Gummistopfen verstanden werden.

### STAND DER TECHNIK

Aus dem Stand der Technik, beispielweise aus der EP 2 711 080 A, sind Hohlnadeln bekannt, welche zum Durchstossen des Verschlusses eines Probengefässes ein zylindrisches Hohlprofil mit einem ersten Abschnitt und mit einem zweiten Abschnitt umfasst. Der erste Abschnitt umfasst die Spitze der Hohlnadel und der zweite Abschnitt weist einen grösseren Durchmesser auf als der erste Abschnitt. Ein konischer Bereich verbindet den ersten Abschnitt mit dem zweiten Abschnitt. Die Spitze der Hohlnadel umfasst eine Schnittkante, mit welcher der Verschluss über die Breite des ersten Abschnitts geschnitten wird.

Dies hat den Nachteil, dass sich benötigte Durchstosskraft erhöht, sobald der zweite Abschnitt mit dem grösseren Durchmesser durch den Verschluss gestossen werden soll. Beim Herausziehen können die durch den Verschluss auf die Hohlnadel wirkenden Klemmkräfte so gross sein, dass der Verschluss vom Probengefäss gezogen wird oder dass der Verschluss zusammen mit dem Probengefäss aus dem Probenhalter gezogen werden. Die erhöhte Durchstosskraft beansprucht zudem die Mechanik des Gerätes zur Laborautomatisierung und führt zu frühzeitiger Abnutzung. Beim Durchstossen des Verschlusses muss zudem gewährleistet sein, dass der Verschluss nur durchstossen wird, nicht jedoch Material des Verschlusses ausgestanzt wird. Das ausgestanzte Material kann entweder die Nadel verstopfen oder die Probe verunreinigen, was beides auf jeden Fall zu vermeiden ist.

Die DE 199 05 644 offenbart eine Doppelhohlnadel mit einer äusseren Kanüle, in welcher eine innere Kanüle angeordnet ist. Dadurch ergeben sich ein erster Abschnitt mit einem kleineren Durchmesser und ein zweiter Abschnitt mit einem grösseren Durchmesser. Durch die abgeschrägte Ausgestaltung des vorderen Endes der äusseren Kanüle ergibt sich eine Schnittkante an dessen Umfang, welche sich beabstandet zur inneren Kanüle um diese erstreckt.

Diese Ausgestaltung hat den Nachteil, dass beim Durchstossen mit der inneren Kanüle eine geringe Kraft nötig ist und beim Durchstossen mit der äusseren Kanüle eine vergleichsweise grössere Kraft.

### BESCHREIBUNG DER ERFINDUNG

Eine Aufgabe der vorliegenden Erfindung besteht darin, eine Hohlnadel bereitzustellen, bei welcher beim Durchstossen, bzw. bei welcher bei der Durchführung durch einen Verschluss, die Klemmkräfte möglichst klein sind.

Diese Aufgabe wird durch eine Hohlnadel mit den Merkmalen des Anspruchs 1 gelöst. Weitere Ausführungsformen der Hohlnadel sind durch Merkmale von weiteren Ansprüchen definiert.

Eine erfindungsgemässe Hohlnadel zum Durchstossen eines Verschlusses eines Probengefässes umfasst ein zylindrisches Hohlprofil, welches sich entlang einer Längsachse erstreckt und an dessen ersten Ende eine Spitze vorgesehen ist. Die Hohlnadel weist einen ersten Abschnitt auf, der die Spitze umfasst und wobei weist einen zweiten Abschnitt auf, der einen grösseren Durchmesser als der erste Abschnitt aufweist. Ein Übergang zwischen dem ersten Abschnitt und dem zweiten Abschnitt umfasst mindestens eine Schnittkante, welche sich vom ersten Abschnitt bis zum zweiten Abschnitt erstreckt, wobei jede Schnittkante durch zwei benachbarte Flanken gebildet wird. Die Schnittkante bewirkt, dass beim Durchstossen des Verschlusses, der Verschluss durch die Schnittkante weiter aufgeschnitten wird, wodurch sich die auf die Hohlnadel seitlich wirkenden Klemmkräfte reduzieren.

In einer Ausführungsform umfasst der Übergang zwei oder mehr Schnittkanten, welche sich vom ersten Abschnitt bis zum zweiten Abschnitt erstrecken.

In einer Ausführungsform sind die Schnittkanten gleichmässig verteilt am Umfang des Übergangs angeordnet. Beispielsweise können zwei sich am Umfang gegenüberliegende Schnittkanten vorgesehen sein oder es können drei Schnittkanten vorgesehen sein, welche um einen Winkel von 120° bezüglich der Längsachse versetzt zueinander angeordnet sind. Grundsätzlich kann eine beliebige Anzahl von Schnittkanten unter beliebigen Winkeln zueinander am Umfang des Übergangs angeordnet sein.

In einer Ausführungsform erstrecken sich die Schnittkanten im Wesentlichen senkrecht zur Längsachse oder die Schnittkanten erstrecken sich unter einem Winkel von weniger als 90° zur Längsachse. Eine zur Längsachse senkrecht ausgerichtete Schnittkante ist einfach herzustellen und eine zur Längsachse winklig angeordnete Schnittkante bewirkt eine kontinuierliche Zunahme der Schnittlänge während der Übergang zwischen dem ersten Abschnitt und dem zweiten Abschnitt der Hohlnadel in den Verschluss eingeführt wird.

In einer Ausführungsform erstrecken sich die beiden benachbarten Flanken unter einem gleichen Winkel zur Längsachse oder unter unterschiedlichen Winkeln zur Längsachse. Beispielsweise erstreckt sich die erste Flanke unter einem ersten Winkel und die zweite Flanke erstreckt sich unter einem zweiten Winkel.

Die beiden Flanken-Winkel können auf die Winkel der Flächen der Spitzen abgestimmt sein. Bei einer Spitze mit asymmetrisch angewinkelten Flächen, ist die erste Fläche der Spitze unter einem ersten Flächen-Winkel ausgerichtet. Die der ersten Fläche gegenüberliegende Fläche der Spitze oder die der ersten Fläche gegenüberliegende resultierende Schnittkante zweier benachbarten Flächen der Spitze ist unter einem Kanten-Winkel angeordnet, der kleiner ist als der Flächen-Winkel. Dementsprechend ist der Winkel der ersten Flanke, welche in der Richtung der Längsachse fluchtend mit der ersten Fläche der Spitze ausgerichtet ist, kleiner als der Winkel der zweiten Flanke, welche mit der zweiten Fläche, bzw. der Kante der Spitze ausgerichtet ist. Ist beispielsweise der erste Flächen-Winkel der Spitze 15° und der gegenüberliegende Kanten-Winkel 4°, so kann der erste Flanken-Winkel 4° sein und der zweite Flanken-Winkel 15°. Dementsprechend wird beim Einführen der Nadelspitze in den Verschluss die Nadel auf eine erste Seite gedrückt. Beim Einführen des Übergangs in den Verschluss wird die Nadel auf die der ersten Seite gegenüberliegende Seite gedrückt. Dementsprechend wird die asymmetrische Belastung der Nadel kompensiert, wodurch die Nadel weniger oder gar nicht ausgelenkt wird. Bei symmetrischen Nadelspitzen können auch die Flanken der Schnittkante symmetrisch ausgebildet sein.

In einer Ausführungsform umfasst die Spitze eine erste Fläche, welche sich unter einem Winkel zur Längsachse über im Wesentlichen den gesamten Querschnitt erstreckt. Diese Bauform ist einfach herzustellen, jedoch wirken auf der Seite der ersten Fläche grössere radiale Kräfte auf die Spitze der Hohlnadel beim Einführen der Hohlnadelspitze in einen Verschluss eines Probengefässes. Alternativ umfasst die Spitze eine erste Fläche und eine zweite Fläche, welche sich unter einem Winkel zur Längsachse über im Wesentlichen die Hälfte des Querschnitts erstrecken. Durch die symmetrische Ausgestaltung der Spitze bezüglich der Längsmittelachse der Hohlnadel, sind die radialen Kräfte, welche beim Einführen in den Verschluss auf die Hohlnadelspitze wirken, symmetrisch bezüglich der Längsmittelachse.

In einer Ausführungsform sind die Flanken der Schnittkante entlang der Längsachse mit den Flächen der Spitze ausgerichtet.

In einer Ausführungsform entspricht ein erster Flanken-Winkel der Schnittkante im Wesentlichen einem Kanten-Winkel der Spitze oder einem zweiten Flächen-Winkel der Spitze, und wobei ein zweiter Flanken-Winkel der Schnittkante im Wesentlichen einem ersten Flächen-Winkel der Spitze entspricht.

In einer Ausführungsform weist die Spitze eine Öffnung auf, welche in der ersten Fläche vorgesehen ist oder die Spitze weist eine Öffnung auf, welche benachbart zur ersten Fläche vorgesehen ist.

In einer Ausführungsform ist im zweiten Abschnitt mindestens eine Ausnehmung vorgesehen, welche sich im Wesentlichen entlang der Längsachse über zumindest einen Bereich des zweiten Abschnitts erstreckt. Die mindestens eine Ausnehmung begrenzt einen Durchgangskanal durch den Verschluss bei einer durch den Verschluss gestossenen Hohlnadel und ermöglicht eine Verbindung des Innern des Probengefässes mit der Umgebung zum Druckausgleich während des Ansaugens respektive des Ausstossens von Flüssigkeit in oder aus der Hohlnadel.

In einer Ausführungsform sind zwei oder mehr Ausnehmungen im zweiten Abschnitt vorgesehen, welche gleichmässig verteilt am Umfang des zweiten Abschnitts angeordnet sind. Beispielsweise können zwei, drei, vier, fünf, sechs oder mehr Ausnehmungen gleichmässig am Umfang verteilt angeordnet sein. Eine unregelmässige Anordnung einer beliebigen Anzahl an Ausnehmungen wäre ebenfalls möglich.

In einer Ausführungsform umfasst die Hohlnadel einen dritten Abschnitt, welcher auf der dem ersten Abschnitt gegenüberliegenden Seite des zweiten Abschnitts an den zweiten Abschnitt anschliesst. Der dritte Abschnitt kann einen grösseren Durchmesser als der zweite Abschnitt aufweisen. Der Durchmesser kann jedoch auch gleichgross sein. Der Übergang zwischen dem zweiten Abschnitt und dem dritten Abschnitt kann stufenförmig oder kontinuierlich ausgestaltet sein.

In einer Ausführungsform umfasst der dritte Abschnitt einen Anschlag, welcher über den Durchmesser des zweiten Abschnitts ragt und welcher zu einem dem ersten Ende der Hohlnadel gegenüberliegenden zweiten Ende beabstandet ist. Der dritte Abschnitt kann weiter einen Konus umfassen, welcher am zweiten Ende ausgebildet ist.

In einer Ausführungsform umfasst die Hohlnadel ein Innenrohr und ein Aussenrohr. Das Innenrohr erstreckt sich über die gesamte Länge der Hohlnadel und die Spitze ist im Innenrohr ausgebildet. Das Aussenrohr erstreckt sich zumindest über einen Bereich des zweiten Abschnitts um das Innenrohr. Die inneren Abmessungen des Aussenrohres sind derart auf die äusseren Abmessungen des Innenrohres abgestimmt, dass das Aussenrohr auf das Innenrohr aufschiebbar ist. Das Aussenrohr kann mit einem beliebigen Verbindungsverfahren mit dem Innenrohr fest verbunden werden, beispielsweise mit Laserschweissen, TIG-Schweissen oder Hartlöten.

In einer Ausführungsform ist die mindestens eine Schnittkante im Aussenrohr ausgebildet. Die Schnittkante kann vor dem Aufschieben des Aussenrohres auf das Innenrohr vorgenommen werden, wodurch sich die Herstellung der Schnittkante wesentlich vereinfacht.

In einer Ausführungsform umfasst die Hohlnadel ein Anschlussrohr, welches sich anschliessend an das Aussenrohr bis zum zweiten Ende der Hohlnadel um das Innenrohr erstreckt. Die inneren Abmessungen des Anschlussrohres sind derart auf die äusseren Abmessungen des Innenrohres abgestimmt, dass das Anschlussrohr auf das Innenrohr aufschiebbar ist. Das Anschlussrohr kann mit einem beliebigen Verbindungsverfahren mit dem Innenrohr fest verbunden werden, beispielsweise mit Laserschweissen, TIG-Schweissen oder Hartlöten.

In einer Ausführungsform ist der Konus, welcher am zweiten Ende der Hohlnadel ausgebildet ist, im Anschlussrohr ausgebildet.

In einer Ausführungsform umfasst die Hohlnadel einen hülsenförmigen Anschlag, der sich über einen Bereich des dritten Abschnitts um das Innenrohr erstreckt. Die inneren Abmessungen des Anschlages sind derart auf die äusseren Abmessungen des Anschlussrohres abgestimmt, dass der Anschlag auf das Anschlussrohr aufschiebbar ist. Der Anschlag kann mit einem beliebigen Verbindungsverfahren mit dem Anschlussrohr fest verbunden werden, beispielsweise mit Laserschweissen, TIG-Schweissen oder Hartlöten.

In einer Ausführungsform umfassen das Innenrohr, das Aussenrohr und das Anschlussrohr ein erstes Material und der Anschlag umfasst ein zweites Material. Alternativ können alle diese Bauteile gleiche oder unterschiedliche Materialien umfassen. Beispielsweise sind beide Materialien CrNi-Stähle. Beispielsweise ist das erste Material X2CrNiMo17-12-2 und das zweite Material X8CrNiS18-9.

Beispielsweise hat das Innenrohr einen Innendurchmesser von 0.5 mm, einen Aussendurchmesser von 0.8 mm und eine Länge von 155 mm. Der Innendurchmesser kann beispielsweise in einem Bereich von 0.3 bis 0.7 mm liegen und der Aussendurchmesser in einem Bereich von 0.6 bis 0.8 mm. Beispielsweise hat das Aussenrohr einen Innendurchmesser von 0.8 mm, einen Aussendurchmesser von 1.6 mm und eine Länge von 111 mm. Der Innendurchmesser kann beispielsweise in einem Bereich von 0.6 bis 0.8 mm liegen und der Aussendurchmesser in einem Bereich von 1.4 bis 2.0 mm. Beispielsweise hat das Anschlussrohr einen Innendurchmesser von 0.8 mm, einen Aussendurchmesser von 2 mm und eine Länge von 34 mm. Beispielsweise hat der Anschlag einen Innendurchmesser von 2 mm, einen Aussendurchmesser von 4 mm und eine Länge von 4 mm. Beispielsweise ist die erste Flanke der Schnittkante des Übergangs unter einem Winkel von 15° bezüglich der Längsachse ausgerichtet und die zweite Flanke unter einem Winkel von 4°. Beispielsweise betragen die Länge der Ausnehmungen im Aussenrohr 83 mm und deren Breite und deren Tiefe betragen 0.3 mm. Bei der Spitze einer asymmetrischen ersten Ausführungsform beträgt beispielsweise der Flächen-Winkel, unter welchem die erste Fläche der Spitze bezüglich der Längsachse ausgerichtet ist 15° und der Kanten-Winkel, der dritten Kante, welche der ersten Fläche gegenüberliegend angeordnet ist 4°. Der Flächen-Winkel kann beispielsweise 10° bis 20° betragen und der Kanten-Winkel 2° bis 6°. Bei der Spitze einer symmetrischen zweiten Ausführungsform beträgt beispielsweise der Flächen-Winkel der ersten Fläche und der zweiten Fläche 15°. Die Flächen-Winkel können beispielsweise 10° bis 20° betragen.

In einer Ausführungsform umfasst die Spitze mindestens eine Schnittkante und die mindestens eine Schnittkante des Übergangs ist mit der mindestens einen Schnittkante der Spitze ausgerichtet. Wenn die Spitze zwei oder mehr Schnittkanten umfasst, so umfasst der Übergang die gleiche Anzahl an Schnittkanten und jede der Schnittkanten des Übergangs ist mit einer entsprechenden Schnittkante der Spitze ausgerichtet. Ausgerichtet heisst hier, dass sie entlang der Längsachse fluchtend miteinander sind.

Die erwähnten Ausführungsformen der Hohlnadel lassen sich in beliebiger Kombination einsetzen, sofern sie sich nicht widersprechen.

### KURZE BESCHREIBUNG DER FIGUREN

Ausführungsbeispiele der vorliegenden Erfindung werden nachstehend anhand von Figuren noch näher erläutert. Diese dienen lediglich zur Erläuterung und sind nicht einschränkend auszulegen. Es zeigen
Fig. 1 eine Seitenansicht einer erfindungsgemässen Hohlnadel;
Fig. 2 eine Schnittansicht durch die Hohlnadel von Figur 1;
Fig. 3A eine Ansicht des Details V der Figur 1;
Fig. 3B eine Ansicht des Details X der Figur 2;
Fig. 3C eine Rückseitige Ansicht der Figur 3A;
Fig. 4 eine Ansicht des Details Y der Figur 2;
Fig. 5 eine Ansicht des Schnitts B-B der Figur 2;
Fig. 6A eine Ansicht des Details W der Figur 3C;
Fig. 6B eine Detailansicht der Spitze der Figur 3B;
Fig. 6C eine Rückseitige Ansicht der Figur 6A;
Fig. 7A ein Schnittbild einer Hohlnadel des Standes der Technik;
Fig. 7B ein Schnittbild einer erfindungsgemässen Hohlnadel;
Fig. 8A eine Detailansicht einer alternativen Hohlnadelspitze;
Fig. 8B eine Schnittansicht der Hohlnadelspitze der Figur 8A;
Fig. 8C eine Rückseitige Ansicht der Figur 8A;
Fig. 9A ein Schnittbild einer Hohlnadel des Standes der Technik;
Fig. 9B ein Schnittbild einer erfindungsgemässen Hohlnadel; und
Fig. 10 die Schritte zum Einsetzen einer erfindungsgemässen Hohlnadel in einen Probenpipettor.

### DETAILLIERTE BESCHREIBUNG DER ERFINDUNG

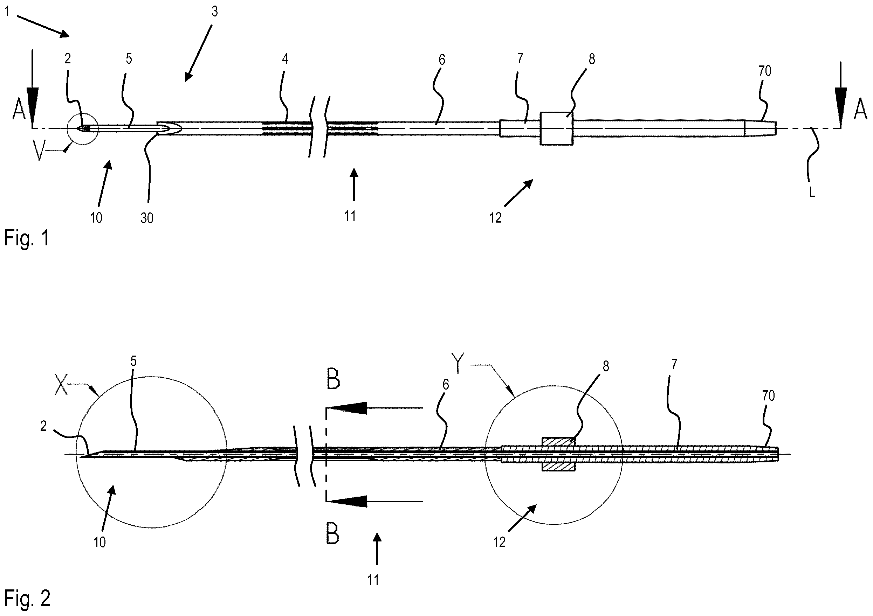

Die Figur 1 zeigt eine Seitenansicht einer erfindungsgemässen Hohlnadel und die Figur 2 eine Schnittansicht durch die Hohlnadel von Figur 1. Die Hohlnadel 1 umfasst ein zylindrisches Hohlprofil, welches sich entlang einer Längsachse L) erstreckt und an dessen ersten Ende eine Spitze 2 vorgesehen ist. Das Hohlprofil kann einen kreisförmigen, ovalen oder n-eckigen Querschnitt aufweisen, wobei n gleich oder grösser oder gleich drei ist. Der n-eckige Querschnitt kann ein regelmässiges oder ein unregelmässiges n-Eck sein. Die Hohlnadel 1 umfasst einen ersten Abschnitt 10, der die Spitze 2 umfasst. Die Hohlnadel 1 umfasst weiter einen zweiten Abschnitt 11, der einen grösseren Durchmesser als der erste Abschnitt 10 aufweist. Ein Übergang 3 zwischen dem ersten Abschnitt 10 und dem zweiten Abschnitt 11 umfasst mindestens eine Schnittkante 30, welche sich vom ersten Abschnitt 10 bis zum zweiten Abschnitt 11 erstreckt. In der dargestellten Ausführungsform umfasst die Hohlnadel 1 weiter einen dritten Abschnitt 12 welcher an den zweiten Abschnitt 11 anschliesst und sich gegenüberliegend dem ersten Ende bis zum zweiten Ende der Hohlnadel erstreckt. Die Länge des ersten Abschnitts 10 ist ein Vielfaches des Durchmessers des ersten Abschnitts 10 und die Länge des zweiten Abschnitts 11 ist ein Vielfaches des Durchmessers des zweiten Abschnitts 11 und ist ein Vielfaches der Länge des ersten Abschnitts 10. Die Länge des dritten Abschnitts 12 ist ein Vielfaches der Länge des ersten Abschnitts 10 und ein Bruchteil der Länge des zweiten Abschnitts 11. In einem mittleren Bereich des zweiten Abschnitts 11 sind mehrere Ausnehmungen 4 gleichmässig am Umfang verteilt angeordnet.

Das gegen die Spitze 2 gerichtete Ende der Ausnehmungen 4 ist beabstandet zur Schnittkante 30 und des der Spitze 2 abgewandte Ende der Ausnehmung 4 ist beabstandet zum dritten Abschnitt 12. Der dritte Abschnitt 12 weist einen nur geringfügig grösseren Durchmesser auf als der zweite Abschnitt 11. Der dritte Abschnitt 12 umfasst ein Anschlussrohr 7, welches sich vom zweiten Abschnitt 11 bis zum zweiten Ende der Hohlnadel 1 erstreckt. Im dritten Abschnitt 12 ist am zweiten Ende der Hohlnadel 1 ein Konus 70 ausgebildet. Der dritte Abschnitt 12 umfasst ein Anschlag 8, dessen Durchmesser grösser ist als derjenige des Anschlussrohres 7. Die Breite des Anschlags 8 entspricht im Wesentlichen seiner Länge. Der Abstand des Anschlags 8 zum zweiten Ende der Hohlnadel 1 ist ein Vielfaches der Länge des Anschlags 8. Die Hohlnadel 1 umfasst ein Innenrohr 5, welches sich vom ersten Ende, d.h. von der Spitze 2 bis zum zweiten Ende, d.h. bis zum Konus 70 erstreckt. Die Spitze 2 umfasst das erste Ende des Innenrohres 5. Die Hohlnadel 1 umfasst weiter ein Aussenrohr 6, welches sich im zweiten Abschnitt 11 um das Innenrohr 5 und an dieses anliegend erstreckt. Die Ausnehmungen 4 sind im Aussenrohr 6 ausgebildet. Die Hohlnadel 1 umfasst weiter ein Anschlussrohr 7, welches sich im dritten Abschnitt 12 um das Innenrohr 5 und an dieses anliegend erstreckt. Der Anschlag 8 ist als Hülse ausgebildet, welche sich um das Anschlussrohr 7 und an dieses anliegend erstreckt. Das Innenrohr 5, das Aussenrohr, 6, das Anschlussrohr 7 und der Anschlag 8 sind durch Laserschweissen fest miteinander verbunden.

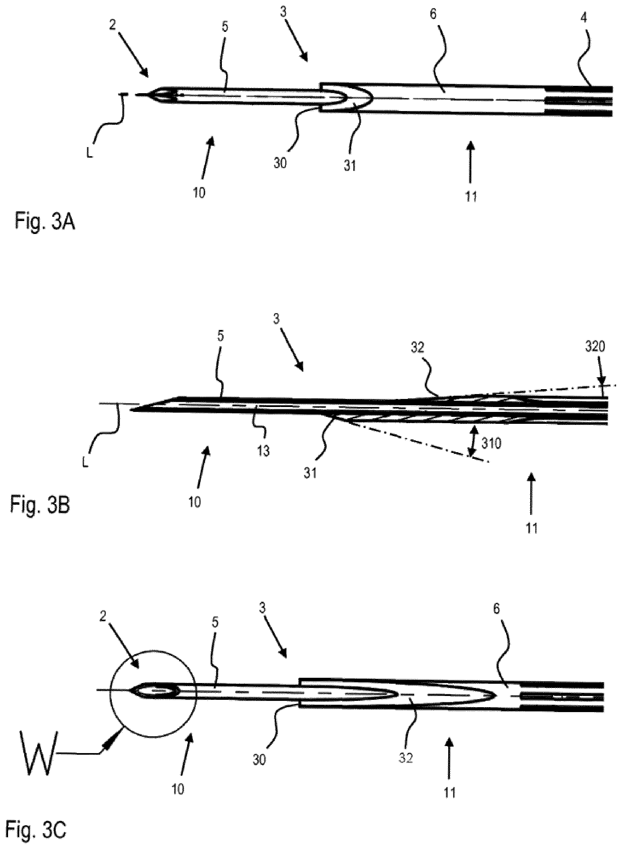

Die Figur 3A zeigt eine Ansicht des Details V der Figur 1, die Figur 3B zeigt eine Ansicht des Details X der Figur 2 und die Figur 3C zeigt eine Rückseitige Ansicht der Figur 3A. Dargestellt ist der erste Abschnitt 10 und ein Teil des zweiten Abschnitts 11 mit einem Teil der Ausnehmungen 4. Vom Übergang 3 ist die Schnittkante 30 und eine erste Flanke 31 sichtbar. Die Schnittkante 30 erstreckt sich im Wesentlichen senkrecht zur Längsachse L der Hohlnadel. Die erste Flanke 31 wird durch ein schräges Anschleifen des Aussenrohres 6 vor dem Zusammenbau mit dem Innenrohr 5 hergestellt. Im Innern des Innenrohres 5 erstreckt sich ein Hohlkanal 13 über dessen gesamte Länge. Die erste Flanke 31 erstreckt sich unter einem ersten Winkel 310 von der Schnittkante 30 schräg in der Richtung des zweiten Endes der Hohlnadel 1. Eine zweite Flanke 32 erstreckt sich unter einem zweiten Winkel 320 von der Schnittkante 30 schräg in der Richtung des zweiten Endes der Hohlnadel 1. In dieser Darstellung ist der erste Winkel 310 ein Vielfaches des zweiten Winkels 320. Die gegen den Übergang 3 gerichteten Enden der Ausnehmungen 4 sind beabstandet zur gegen das zweite Ende der Hohlnadel 1 gerichteten Kante der ersten und der zweiten Flanke 31,32.

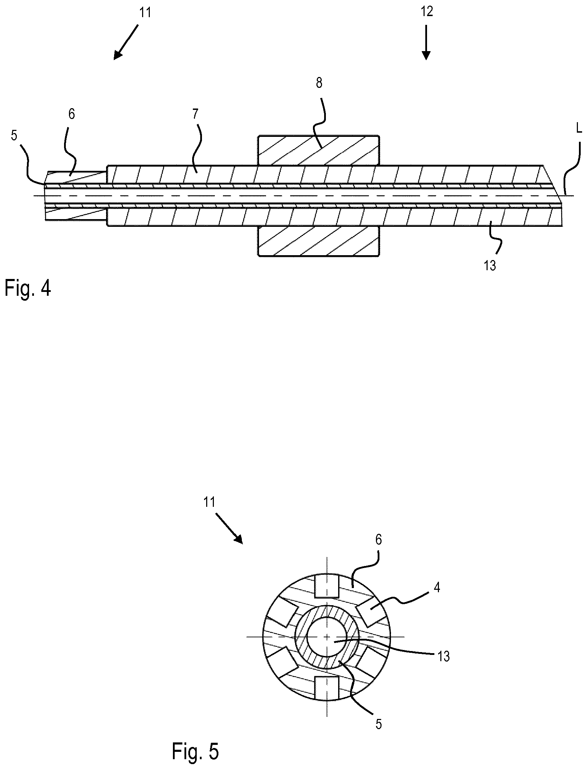

Die Figur 4 zeigt eine Ansicht des Details Y der Figur 2. Das Innenrohr 5 mit dem Hohlkanal 13 erstreckt sich entlang der Längsachs L. Das Aussenrohr 6 umgibt das Innenrohr 5 im zweiten Abschnitt 11 und das Anschlussrohr 7 umgibt das Innenrohr 5 im dritten Abschnitt 12. Das Anschlussrohr 7 schliesst bündig an das Aussenrohr 6 an. Der Anschlag 8 umgibt einen Bereich des Anschlussrohres 7. Die Wandstärke des Anschlags 8 ist grösser als die Wandstärke des Anschlussrohres 7.

Die Figur 5 zeigt eine Ansicht des Schnitts B-B der Figur 2. Dargestellt ist ein Schnitt durch den Bereich der Ausnehmungen 4 im zweiten Abschnitt 11 der Hohlnadel 1. Sechs Ausnehmungen 4 sind gleichmässig verteilt am Umfang des Aussenrohres 6 angeordnet. Die Tiefe der Ausnehmungen 4 ist grösser als die Hälfte der Wandstärke des Aussenrohres 6. Die Wandstärke des Aussenrohres 6 ist ein Vielfaches der Wandstärke des Innenrohres 5.

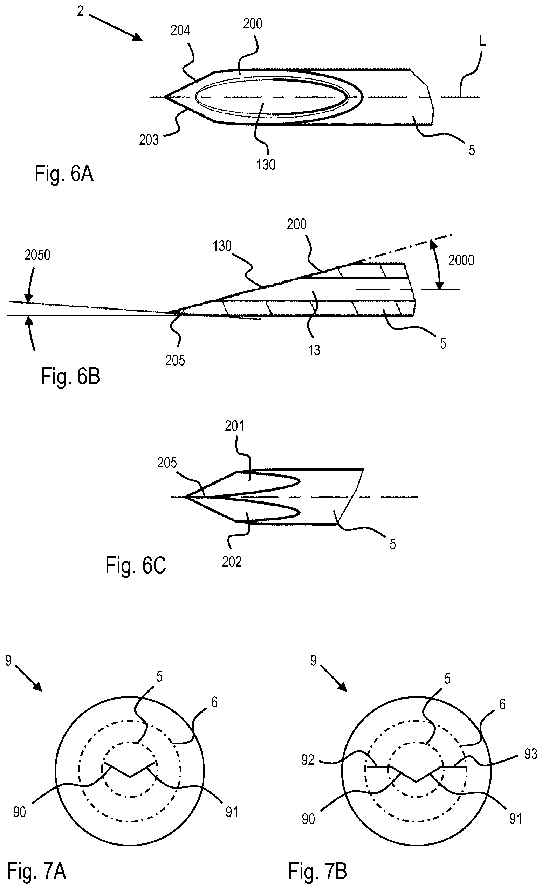

Die Figur 6A zeigt eine Ansicht des Details W der Figur 3C, die Figur 6B zeigt eine Detailansicht der Spitze der Figur 3B und die Figur 6C zeigt eine Rückseitige Ansicht der Figur 6A. Die Spitze 2 der Hohlnadel 1 umfasst eine erste Fläche 200, welche sich unter einem Flächen-Winkel 2000 bezüglich der Längsachse L erstreckt. Die erste Fläche 200 kann durch Abschleifen des Innenrohres 5 erzeugt werden. Auf der bezüglich der Längsachse L gegenüberliegenden Seite der ersten Fläche 200 sind eine zweite Fläche 201 und eine dritte Fläche 202 vorgesehen, wobei die zweite Fläche 201 zur ersten Fläche 200 und zur dritten Fläche 202 winklig angeordnet ist. Eine erste Kannte 203 wird von der Schnittlinie der ersten Fläche 200 mit der zweiten Fläche 201 gebildet, eine zweite Kante 204 wird von der Schnittline der ersten Fläche 200 mit der dritten Fläche 202 gebildet und eine dritte Kante 205 wird von der Schnittlinie der zweiten Fläche 201 mit der dritten Fläche 203 gebildet. Die dritte Kante 205 erstreckt sich unter einem Kanten-Winkel 2050 bezüglich der Längsachse. Wenn die Spitze 2 in den Verschluss eingeführt wird, wirken von der Seite der ersten Fläche 200 erste radiale Kräfte auf die Hohlnadel 1. Die Ausgestaltung der zweiten Fläche 201, der dritten Fläche 202, zusammen mit der dritten Kante 205 erzeugen zweite radiale Kräfte in einer zur Richtung der ersten Kräfte entgegengesetzten Richtung. Die Spitze 2 der Hohlnadel 1 wird dementsprechend weniger zur Seite gedrückt, wodurch die Hohlnadel zentrierter in ein Probengefäss einführbar ist. Der Hohlkanal 13 erstreckt sich durch das gesamte Innenrohr 5 und mündet in einer Öffnung 130, welche von der ersten Fläche 200 umfasst ist.

Die Figur 7A zeigt ein Schnittbild 9 einer Hohlnadel des Standes der Technik, wie es entsteht, wenn eine Spitze, wie sie in den Figuren 6A bis 6C dargestellt ist, durch einen Verschluss gestossen wird. Durch die erste Schnittkante 203 wird ein erster Abschnitt 90 in den Verschluss geschnitten und durch die zweite Schnittkante 204 ein zweiter Abschnitt 91. Die äusseren Enden des ersten und des zweiten Abschnitts 90,91 liegen auf einem Durchmesser, welcher demjenigen des ersten Abschnitts 10, bzw. des Innenrohres 5 entspricht. Soll der zweite Abschnitt 11, bzw. das Aussenrohr 6 durch den Verschluss gestossen werden, müssen die Abschnitte 90,91 aufgeweitet werden, wodurch radiale Klemmkräfte entstehen, welche auf die Hohlnadel wirken. Durch die erhöhten radialen Kräfte erhöht sich auch die Kraft, welche benötigt wird, um die Hohlnadel durch den Verschluss zu stossen, bzw. um die Hohlnadel aus dem Verschluss zu ziehen.

Die Figur 7B zeigt ein Schnittbild einer erfindungsgemässen Hohlnadel, wie es entsteht, wenn zuerst die Spitze gemäss den Figuren 6A bis 6C durch den Verschluss gestossen wird und anschliessend der Übergang 3 mit zwei sich am Umfang gegenüberliegenden Schnittkanten 30, wie sie in der Figur 3A dargestellt sind. Durch die Spitze 2 werden wieder ein erster Abschnitt 90 und ein zweiter Abschnitt 91 erzeugt. Wird der Übergang 3 durch den Verschluss gestossen, so erzeugen die Schnittkanten 30 einen dritten Abschnitt 92, welcher an den ersten Abschnitt 90 anschliesst und einen vierten Abschnitt 93, welcher an den zweiten Abschnitt 91 anschiesst. Die äusseren Enden des dritten und des vierten Abschnitts 92,93 liegen auf einem Durchmesser, welcher demjenigen des zweiten Abschnitts 11, bzw. des Aussenrohres 6 entspricht. Wird der Übergang 3 durch den Verschluss gestossen, so entstehen durch dessen Schnittkanten 30 Schnittkräfte, diese sind jedoch kleiner als die zur Aufweitung des Verschlusses benötigten Kräfte, wenn keine Schnittkanten 30 vorhanden sind. Dadurch, dass sich alle Abschnitte 90,91,92,93 zusammen über im Wesentlichen den Durchmesser des zweiten Abschnitts erstrecken, sind die Kräfte viel kleiner, welche für die Aufweitung des Verschlusses erforderlich sind.

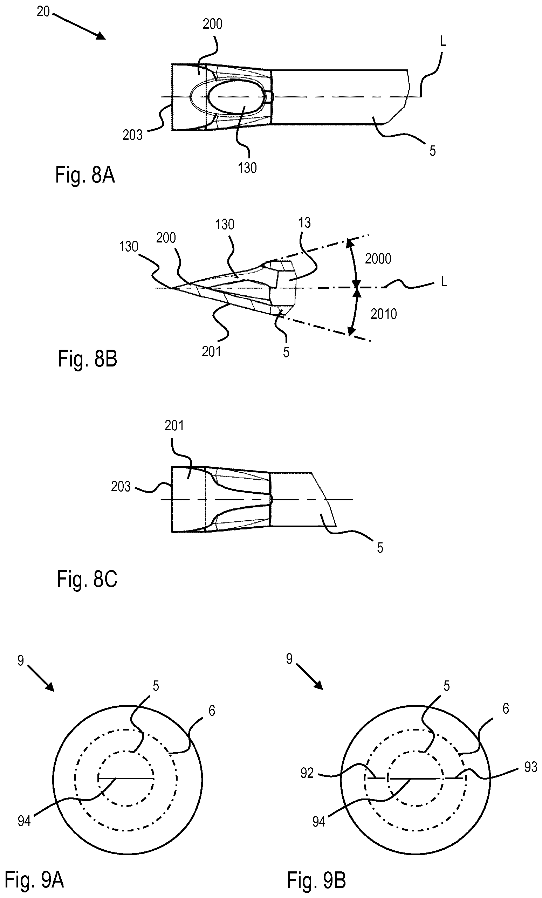

Die Figur 8A zeigt eine Detailansicht einer alternativen Hohlnadelspitze 20, die Figur 8B zeigt eine Schnittansicht der Hohlnadelspitze 20 der Figur 8A und die Figur 8C zeigt eine Rückseitige Ansicht der Figur 8A. In dieser Ausführungsform wird der erste Abschnitt 10, bzw. das Innenrohr 5 zusammengedrückt und anschliessend von zwei Seiten symmetrisch bezüglich der Längsachse L abgeschliffen. Dabei entstehen eine erste Fläche 200 und eine zweite Fläche 201. Die erste Fläche 200 ist unter einem ersten Flächen-Winkel 2000 bezüglich der Längsachse L ausgerichtet und die zweite Fläche 201 unter einem zweiten Flächen-Winkel 2010. Die Schnittlinie der ersten Fläche 200 mit der zweiten Fläche 201 ergibt die erste Kante 203. Alternativ könnten zwei erste Flächen 200 und zwei zweite Flächen 201 in die Spitze 20 geschliffen werden, wobei die zwei ersten Flächen 200 winklig zueinander ausgerichtet sind und wobei die zwei zweiten Flächen 201 winklig zueinander ausgerichtet sind. Dadurch entstehen zwei erste Kanten 203, welche winklig zueinander ausgerichtet sind. Eine symmetrische Anordnung und Ausrichtung der ersten und zweiten Flächen resultieren in einer symmetrischen Anordnung und Ausrichtung der ersten Kanten. In der dargestellten Spitze 20 mündet der Hohlkanal 13 in eine Öffnung 130, welche benachbart zur ersten Fläche 200 angeordnet ist.

Die Figur 9A zeigt ein Schnittbild einer Hohlnadel des Standes der Technik. Durch die in den Figuren 8A bis 8C dargestellte Spitze 20 wird nur ein zentraler fünfter Abschnitt 94 in den Verschluss geschnitten. Der fünfte Abschnitt 94 erstreckt sich im Wesentlichen über den Durchmesser des ersten Abschnitts 10, bzw. des Innenrohres 5. Durch das Zusammendrücken des Innenrohres 5 wird die Spitze 20 an ihrem Ende aufgeweitet, wodurch die erster Schnittkante 203 über den Durchmesser des Innenrohres 5 ragt.

Die Figur 9B zeigt ein Schnittbild einer erfindungsgemässen Hohlnadel. Durch die Schnittkanten 30 des Übergangs 3 werden ein dritter Abschnitt 92 und ein vierter Abschnitt 93 in den Verschluss geschnitten, wobei der dritte und der vierte Abschnitt 92,93 seitlich an den zentrischen fünften Abschnitt 94 anschliessen und fluchtend mit diesem ausgerichtet sind.

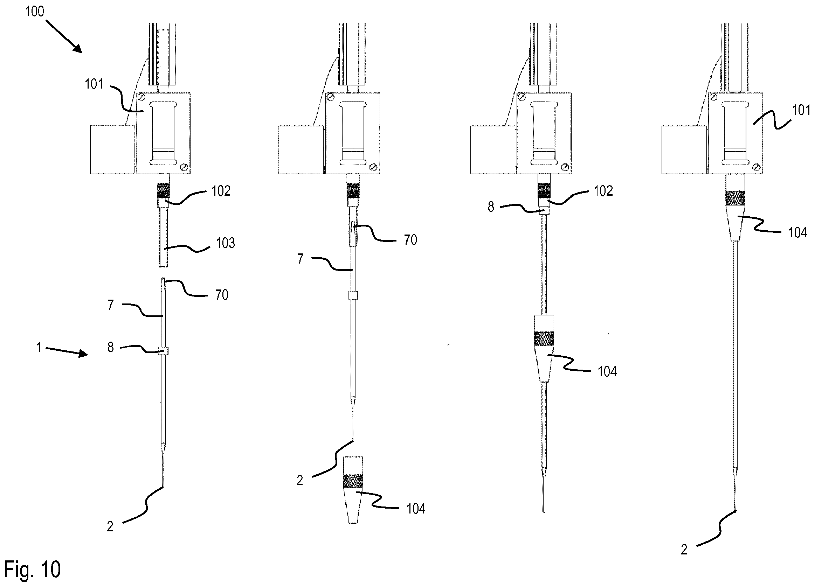

Die Figur 10 zeigt die Schritte zum Einsetzen einer erfindungsgemässen Hohlnadel 1 in einen Probenpipettor 100. Der Probenpipettor 100 umfasst eine Halterung 101 mit einer Anschlusshülse 102, in welcher ein Schlauch 103 verschiebbar gelagert ist und eine Überwurfmutter 104, mit welcher die Hohlnadel an der Halterung 101 befestigbar ist. Zum Pipettieren kann im Schlauch 103 ein Unterdruck oder ein Überdruck erzeugt werden. Die Halterung 101 ist in der Horizontalen, sowie in der Vertikalen vefahrbar. Das Anschlussrohr 7 der Hohlnadel 1 wird mit dem Konus 70 in den Schlauch 103 eingeschoben. Der Schlauch 103 wird zusammen mit der Hohlnadel 1 bis zum Anschlag 8 in die Anschlusshülse 102 geschoben. Die Überwurfmutter 104 wird von der Spitze 2 über die Hohlnadel 1 bis zum Anschlag 8 geschoben und die Überwurfmutter 104 wird auf der Anschlusshülse 102 festgeschraubt.

**BEZUGSZEICHENLISTE**

| | | | |
|---|---|---|---|
| 1 | Hohlnadel | 32 | zweite Flanke |
| 10 | erster Abschnitt | 320 | zweiter Winkel |
| 11 | zweiter Abschnitt | 4 | Ausnehmung |
| 12 | dritter Abschnitt | 5 | Innenrohr |
| 13 | Hohlkanal | 6 | Aussenrohr |
| 130 | Öffnung | 7 | Anschlussrohr |
| 2 | Spitze | 70 | Konus |
| 20 | Spitze | 8 | Anschlag |
| 200 | erste Fläche | 9 | Schnittbild |
| 2000 | erster Flächen-Winkel | 90 | erster Abschnitt |
| 201 | zweite Fläche | 91 | zweiter Abschnitt |
| 2010 | zweiter Flächen-Winkel | 92 | dritter Abschnitt |
| | | 93 | vierter Abschnitt |
| 202 | dritte Fläche | 94 | fünfter Abschnitt |
| 203 | erste Kante | 100 | Pipettor |
| 204 | zweite Kante | 101 | Halterung |
| 205 | dritte Kante | 102 | Anschluss |
| 2050 | Kanten-Winkel | 103 | Schlauch |
| 3 | Übergang | 104 | Überwurfmutter |
| 30 | Schnittkante | | |
| 31 | erste Flanke | L | Längsachse |
| 310 | erster Winkel | | |

## Patentansprüche

1. Eine Hohlnadel (1) zum Durchstossen eines Verschlusses eines Probengefässes, wobei die Hohlnadel (1) ein zylindrisches Hohlprofil umfasst, welches sich entlang einer Längsachse (L) erstreckt und an dessen erstem Ende eine Spitze (2;20) vorgesehen ist, wobei die Hohlnadel (1) einen ersten Abschnitt (10) aufweist, der die Spitze (2;20) umfasst und wobei die Hohlnadel (1) einen zweiten Abschnitt (11) aufweist, der einen grösseren Durchmesser als der erste Abschnitt (10) aufweist,
**dadurch gekennzeichnet, dass** ein Übergang (3) zwischen dem ersten Abschnitt (10) und dem zweiten Abschnitt (11) mindestens eine Schnittkante (30) umfasst, welche sich vom ersten Abschnitt (10) bis zum zweiten Abschnitt (11) erstreckt, wobei jede Schnittkante (30) durch zwei dazu benachbarte Flanken (31,32) gebildet wird.

2. Die Hohlnadel (1) gemäss Anspruch 1, wobei der Übergang (3) zwei oder mehr Schnittkanten (30) umfasst, welche sich vom ersten Abschnitt (10) bis zum zweiten Abschnitt (11) erstrecken.

3. Die Hohlnadel (1) gemäss Anspruch 1 oder 2, wobei die Schnittkanten (30) gleichmässig verteilt am Umfang des Übergangs (3) angeordnet sind.

4. Die Hohlnadel (1) gemäss einem der vorangehenden Ansprüche, wobei sich die Schnittkanten (30) im Wesentlichen senkrecht zur Längsachse (L) erstrecken oder wobei sich die Schnittkanten (30) unter einem Winkel von weniger als 90° zur Längsachse (L) erstrecken.

5. Die Hohlnadel (1) gemäss einem der vorangehenden Ansprüche, wobei sich die beiden benachbarten Flanken (31, 32) unter einem gleichen Winkel zur Längsachse (L) erstrecken oder sich die beiden benachbarten Flanken (31, 32) unter unterschiedlichen Winkeln zur Längsachse (L) erstrecken.

6. Die Hohlnadel (1) gemäss einem der vorangehenden Ansprüche, wobei die Spitze (2) eine erste Fläche (200) umfasst, welche sich unter einem Winkel zur Längsachse (L) über im Wesentlichen den gesamten Querschnitt erstreckt oder wobei die Spitze (20) eine erste Fläche (200) und eine zweite Fläche (201) umfasst, welche sich unter einem Winkel zur Längsachse (L) über im Wesentlichen die Hälfte des Querschnitts erstrecken.

7. Die Hohlnadel gemäss Anspruch 6, wobei die Flanken (31, 32) der Schnittkante (30) entlang der Längsachse (L) mit den Flächen (200,201) der Spitze (2;20) ausgerichtet sind.

8. Die Hohlnadel gemäss Anspruch 7, wobei ein erster Flanken-Winkel (310) der Schnittkante (30) im Wesentlichen einem Kanten-Winkel (2050) der Spitze (2) oder einem zweiten Flächen-Winkel (2010) der Spitze (20) entspricht und wobei ein zweiter Flanken-Winkel (320) der Schnittkante (30) im Wesentlichen einem ersten Flächen-Winkel (2000) der Spitze (2;20) entspricht.

9. Die Hohlnadel (1) gemäss Anspruch 6, wobei die Spitze (2; 20) eine Öffnung (130) aufweist, welche in der ersten Fläche (200) vorgesehen ist oder welche benachbart zur ersten Fläche (200) vorgesehen ist.

10. Die Hohlnadel (1) gemäss einem der vorangehenden Ansprüche, wobei im zweiten Abschnitt (11) mindestens eine Ausnehmung (4) vorgesehen ist, welche sich im Wesentlichen entlang der Längsachse (L) über zumindest einen Bereich des zweiten Abschnitts (11) erstreckt.

11. Die Hohlnadel (1) gemäss Anspruch 10, wobei zwei oder mehr Ausnehmungen (4) im zweiten Abschnitt (11) vorgesehen sind, welche gleichmässig verteilt am Umfang des zweiten Abschnitts (11) angeordnet sind.

12. Die Hohlnadel (1) gemäss einem der vorangehenden Ansprüche, wobei die Hohlnadel (1) einen dritten Abschnitt (12) umfasst, welcher auf der dem ersten Abschnitt (10) gegenüberliegenden Seite des zweiten Abschnitts (11) an den zweiten Abschnitt (11) anschliesst.

13. Die Hohlnadel (1) gemäss Anspruch 12, wobei der dritte Abschnitt (12) einen Anschlag (6) umfasst, welcher über den Durchmesser des zweiten Abschnitts (11) ragt und welcher zu einem dem ersten Ende der Hohlnadel (1) gegenüberliegenden zweiten Ende beabstandet ist.

14. Die Hohlnadel (1) gemäss einem der vorangehenden Ansprüche, wobei die Hohlnadel (1) ein Innenrohr (5) und ein Aussenrohr (6) umfasst, wobei sich das Innenrohr (5) über die gesamte Länge der Hohlnadel (1) erstreckt und die Spitze (2;20) im Innenrohr (5) ausgebildet ist, und wobei sich das Aussenrohr (6) zumindest über einen Bereich des zweiten Abschnitts (11) um das Innenrohr (5) erstreckt.

15. Die Hohlnadel (1) gemäss Anspruch 14, wobei die mindestens eine Schnittkante (30) im Aussenrohr (6) ausgebildet ist.

16. Die Hohlnadel (1) gemäss Anspruch 14 oder 15, wobei die Hohlnadel (1) ein Anschlussrohr (7) umfasst, welches sich anschliessend an das Aussenrohr (8) bis zum zweiten Ende der Hohlnadel (1) um das Innenrohr (5) erstreckt.

17. Die Hohlnadel (1) gemäss einem der vorangehenden Ansprüche, wobei die Spitze (2;20) mindestens eine Schnittkante (203;204) umfasst und die mindestens eine Schnittkante (30) des Übergangs (3) mit der mindestens einen Schnittkante (203;204) der Spitze (2;20) ausgerichtet ist.

## Claims

1. A hollow needle (1) for piercing a closure of a sample container, said hollow needle (1) comprising a cylindrical hollow profile extending along a longitudinal axis (L) and having a tip (2;20) provided at a first end thereof, said hollow needle (1) having a first section (10) comprising said tip (2;20) and said hollow needle (1) having a second section (11) having a larger diameter than said first section (10),
**characterized in that** a transition (3) between the first section (10) and the second section (11) comprises at least one cutting edge (30) extending from the first section (10) to the second section (11), wherein each cutting edge (30) being formed by two flanks (31,32) adjacent thereto.

2. The hollow needle (1) according to claim 1, wherein the transition (3) comprises two or more cutting edges (30) extending from the first section (10) to the second section (11).

3. The hollow needle (1) according to claim 1 or 2, wherein the cutting edges (30) are evenly distributed around the circumference of the transition (3).

4. The hollow needle (1) according to any one of the preceding claims, wherein the cutting edges (30) extend substantially perpendicular to the longitudinal axis (L) or wherein the cutting edges (30) extend at an angle of less than 90° to the longitudinal axis (L).

5. The hollow needle (1) according to any one of the preceding claims, wherein the two adjacent flanks (31,32) extend at an equal angle to the longitudinal axis (L) or wherein the two adjacent flanks (31,32) extend at different angles to the longitudinal axis (L).

6. The hollow needle (1) according to any one of the preceding claims, wherein the tip (2) comprises a first surface (200) extending at an angle to the longitudinal axis (L) over substantially the entire cross-section, or wherein the tip (20) comprises a first surface (200) and a second surface (201) extending at an angle to the longitudinal axis (L) over substantially half of the cross-section.

7. The hollow needle according to claim 6, wherein the flanks (31,32) of the cutting edge (30) are aligned along the longitudinal axis (L) with the faces (200,201) of the tip (2; 20).

8. The hollow needle according to claim 7, wherein a first flank angle (310) of the cutting edge (30) substantially corresponds to an edge angle (2050) of the tip (2) or a second face angle (2010) of the tip (20), and wherein a second flank angle (320) of the cutting edge (30) substantially corresponds to a first face angle (2000) of the tip (2; 20).

9. The hollow needle (1) according to claim 6, wherein the tip (2;20) comprises an opening (130) provided in the first surface (200) or provided adjacent to the first surface (200).

10. The hollow needle (1) according to any one of the preceding claims, wherein at least one recess (4) is provided in the second section (11), which recess extends substantially along the longitudinal axis (L) over at least a portion of the second section (11).

11. The hollow needle (1) according to claim 10, wherein two or more recesses (4) are provided in the second section (11), which are evenly distributed around the circumference of the second section (11).

12. The hollow needle (1) according to any one of the preceding claims, wherein the hollow needle (1) comprises a third section (12) which joins the second section (11) on the side of the second section (11) opposite to the first section (10).

13. The hollow needle (1) according to claim 12, wherein the third section (12) comprises a stop (6) which protrudes beyond the diameter of the second section (11), and which is spaced apart from a second end of the hollow needle (1), opposite to its first end.

14. The hollow needle (1) according to any one of the preceding claims, wherein the hollow needle (1) comprises an inner tube (5) and an outer tube (6), wherein the inner tube (5) extends over the entire length of the hollow needle (1) and the tip (2;20) is formed in the inner tube (5), and wherein the outer tube (6) extends over at least a portion of the second section (11) around the inner tube (5) .

15. The hollow needle (1) according to claim 14, wherein the at least one cutting edge (30) is formed in the outer tube (6).

16. The hollow needle (1) according to claim 14 or 15, wherein the hollow needle (1) comprises a connecting tube (7) extending around the inner tube (5) adjacent to the outer tube (8) to the second end of the hollow needle (1).

17. The hollow needle (1) according to any one of the preceding claims, wherein the tip (2;20) comprises at least one cutting edge (203;204) and the at least one cutting edge (30) of the transition (3) is aligned with the at least one cutting edge (203;204) of the tip (2;20).

## Revendications

1. Une aiguille creuse (1) pour percer une fermeture d'un récipient d'échantillon, ladite aiguille creuse (1) comprenant un profil creux cylindrique s'étendant le long d'un axe longitudinal (L) et ayant une pointe (2;20) prévue à une première extrémité de celle-ci, ladite aiguille creuse (1) ayant une première section (10) comprenant ladite pointe (2;20) et ladite aiguille creuse (1) ayant une seconde section (11) ayant un diamètre plus grand que ladite première section (10),
**caractérisée en ce qu'**une transition (3) entre la première section (10) et la seconde section (11) comprend au moins une arête de coupe (30) s'étendant de la première section (10) à la seconde section (11), dans laquelle chaque arête de coupe (30) est formée par deux flancs (31,32) adjacents à celle-ci.

2. L'aiguille creuse (1) selon la revendication 1, dans laquelle la transition (3) comprend deux ou plusieurs arêtes de coupe (30) s'étendant de la première section (10) à la seconde section (11).

3. L'aiguille creuse (1) selon la revendication 1 ou 2, dans laquelle les arêtes de coupe (30) sont réparties uniformément autour de la circonférence de la transition (3).

4. L'aiguille creuse (1) selon l'une quelconque des revendications précédentes, dans laquelle les arêtes de coupe (30) s'étendent sensiblement perpendiculairement à l'axe longitudinal (L) ou dans laquelle les arêtes de coupe (30) s'étendent selon un angle inférieur à 90° par rapport à l'axe longitudinal (L).

5. L'aiguille creuse (1) selon l'une quelconque des revendications précédentes, dans laquelle les deux flancs adjacents (31,32) s'étendent selon un angle égal par rapport à l'axe longitudinal (L) ou dans laquelle les deux flancs adjacents (31,32) s'étendent selon des angles différents par rapport à l'axe longitudinal (L).

6. L'aiguille creuse (1) selon l'une quelconque des revendications précédentes, dans laquelle la pointe (2) comprend une première surface (200) s'étendant angulairement par rapport à l'axe longitudinal (L) sur sensiblement toute la section transversale, ou dans laquelle la pointe (20) comprend une première surface (200) et une seconde surface (201) s'étendant angulairement par rapport à l'axe longitudinal (L) sur sensiblement la moitié de la section transversale.

7. L'aiguille creuse selon la revendication 6, dans laquelle les flancs (31,32) de l'arête de coupe (30) sont alignés le long de l'axe longitudinal (L) avec les faces (200,201) de la pointe (2;20).

8. L'aiguille creuse selon la revendication 7, dans laquelle un premier angle de flanc (310) de l'arête de coupe (30) correspond sensiblement à un angle d'arête (2050) de la pointe (2) ou à un second angle de face (2010) de la pointe (20), et dans laquelle un second angle de flanc (320) de l'arête de coupe (30) correspond sensiblement à un premier angle de face (2000) de la pointe (2; 20).

9. L'aiguille creuse (1) selon la revendication 6, dans laquelle la pointe (2;20) comprend une ouverture (130) prévue dans la première surface (200) ou prévue adjacente à la première surface (200).

10. L'aiguille creuse (1) selon l'une quelconque des revendications précédentes, dans laquelle au moins un évidement (4) est prévu dans la seconde section (11) qui s'étend sensiblement selon l'axe longitudinal (L) sur au moins une partie de la seconde section (11).

11. L'aiguille creuse (1) selon la revendication 10, dans laquelle deux ou plusieurs évidements (4) sont prévus dans la seconde section (11), qui sont répartis uniformément autour de la circonférence de la seconde section (11).

12. L'aiguille creuse (1) selon l'une quelconque des revendications précédentes, dans laquelle l'aiguille creuse (1) comprend une troisième section (12) qui rejoint la deuxième section (11) sur le côté de la seconde section (11) opposé à la première section (10).

13. L'aiguille creuse (1) selon la revendication 12, dans laquelle la troisième section (12) comprend une butée (6) qui fait saillie au-delà du diamètre de la seconde section (11), et qui est espacée d'une deuxième extrémité de l'aiguille creuse (1), opposée à sa première extrémité.

14. L'aiguille creuse (1) selon l'une quelconque des revendications précédentes, dans laquelle l'aiguille creuse (1) comprend un tube interne (5) et un tube externe (6), dans laquelle le tube interne (5) s'étend sur toute la longueur de l'aiguille creuse (1) et la pointe (2;20) est formée dans le tube interne (5), et dans laquelle le tube externe (6) s'étend sur au moins une partie de la seconde section (11) autour du tube interne (5).

15. L'aiguille creuse (1) selon la revendication 14, dans laquelle l'au moins une arête de coupe (30) est formée dans le tube extérieur (6).

16. L'aiguille creuse (1) selon la revendication 14 ou 15, dans laquelle l'aiguille creuse (1) comprend un tube de connexion (7) s'étendant autour du tube interne (5) adjacent au tube externe (8) jusqu'à la seconde extrémité de l'aiguille creuse (1).

17. L'aiguille creuse (1) selon l'une quelconque des revendications précédentes, dans laquelle la pointe (2;20) comprend au moins une arête de coupe (203;204) et la au moins une arête de coupe (30) de la transition (3) est alignée avec la au moins une arête de coupe (203;204) de la pointe (2; 20).
